# EUROPEAN PATENT APPLICATION

(11) **EP 4 406 951 A1**
(43) Date of publication of application: **31.07.2024**
(21) Application number: 22882961.0
(22) Date of filing: 21.10.2022
(51) Int. Cl.: C07D 405/14, C07D 401/14, C07D 487/04, A61K 31/497, A61K 31/4427, A61P 3/10, A61P 9/00

(54) **COMPOUND AS GLP-1 RECEPTOR AGONIST AND USE THEREOF**

(30) Priority: 22.10.2021 CN 202111235046
(71) Applicant: CGeneTech (Suzhou, China) Co., Ltd., Suzhou, Jiangsu 215123 (CN)
(72) Inventor: WANG, Tong, Suzhou, Jiangsu 215123 (CN); HAO, Yan, Suzhou, Jiangsu 215123 (CN); TANG, Mulin, Suzhou, Jiangsu 215123 (CN); DING, Juping, Suzhou, Jiangsu 215123 (CN); YU, Qiang, Suzhou, Jiangsu 215123 (CN); CHEN, Bin, Suzhou, Jiangsu 215123 (CN); OUYANG, Zhenwu, Suzhou, Jiangsu 215123 (CN)
(74) Representative: LLR
(86) International application number: PCT/CN2022/126581
(87) International publication number: WO 2023/066356

(57) **Abstract**

The present application provides compounds as shown in Formula (I) and uses thereof. The compounds shown in Formula (I) provided by the present application may serve as effective glucagon-like peptide-1 (GLP-1) receptor agonists. They have excellent GLP-1R receptor agonist activity and significant blood glucose-reducing effect, have better pharmacokinetic characteristics, represent more options for the prevention and/or treatment of GLP-1 activity-related diseases, conditions, or disorders, and thereby have better clinical application prospects.

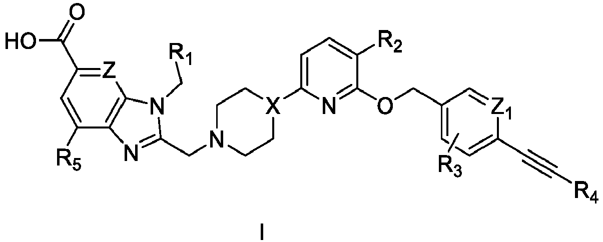

## Description

### Cross-Reference to Related Application

The present application claims priority to a Chinese patent application filed on 22 October, 2021, with an application number 202111235046.8 and an invention title "Compounds as GLP-1 Receptor Agonists and Uses Thereof', the entire contents of which are incorporated herein by reference.

### Technical Field

The present application relates to the field of pharmaceutical chemistry, specifically to compounds as GLP-1 receptor agonists and uses thereof.

### Background Art

Diabetes becomes one of the most important public health problems in current society due to its increasing prevalence and related health risks.

This disease is characterized by hyperglycemia caused by defects in insulin secretion and/or insulin action. Nowadays, two major forms of diabetes are already identified, namely type 1 diabetes and type 2 diabetes mellitus. Type-1 diabetes (T1D) is a class of autoimmune disease that involves multiple immune cells targeting pancreatic β cells. At present, the main treatment for T1D is insulin injection. Although this treatment may effectively control stable blood glucose levels in patients, it may not prevent progressive failure of pancreatic islet function. Type-2 diabetes mellitus (T2DM), also known as non-insulin-dependent diabetes mellitus, is related to insulin resistance and insufficient insulin secretion.

At present, multiple pharmacological methods may be used to treat hyperglycemia and subsequently used to treat T2DM. These methods may be divided into six categories: (A) insulin secretagogues, including sulfonylureas, meglitinides, dipeptidyl peptidase IV (DPP-IV) inhibitors, and glucagon-like peptide-1 receptor (GLP-1R) agonists; (B) biguanides, such as metformin; (C) α-glucosidase inhibitors, such as acarbose; (D) thiazolidinediones, such as pioglitazone; (E) insulin alone or in combination with the above medicaments; and (F) sodium-glucose co-transporter 2 (SGLT2) inhibitors, such as dapagliflozin. Among the above medicaments, except for the GLP-1R agonists and SGLT2 inhibitors, their efficacy is limited and the currently most important problem is not solved yet: β cell function failure and related obesity.

Obesity is a chronic disease that is highly prevalent and associated with many medical issues in modern society, including hypertension, hypercholesterolemia, and cardiovascular and cerebrovascular diseases and the like. In addition, obesity is highly associated with T2DM and insulin resistance, and the latter is often accompanied by hyperinsulinemia and/or hyperglycemia. At present, bariatric surgery is one of the main treatment methods for obesity, but this treatment method is expensive and has risks. Pharmacological interventions are often less effective and have side effects. Therefore, it is apparent that a more effective pharmacological intervention method is needed for obesity, and it has fewer side effects and is convenient for administration.

In addition to hyperglycemia and insulin resistance, T2DM is also associated with the following diseases: hepatic insulin resistance, glucose intolerance, diabetic neuropathy, diabetic nephropathy, diabetic retinopathy, obesity, dyslipidemia, hypertension, hyperinsulinemia, and non-alcoholic fatty liver disease (NAFLD).

The glucagon-like peptide-1 (GLP-1) is an incretin composed of 30 or 31 amino acid residues and secreted by neuroendocrine L cells in the distal ileum and colon in response to food intake. It has already been shown that GLP-1 stimulates insulin secretion, reduces glucagon secretion, inhibits gastric emptying, reduces appetite, and stimulates β cell proliferation in physiological and glucose-dependent ways. In patients with T2DM, the normal postprandial elevation of GLP-1 is absent or decreased (Vilsboll T. et al., Diabetes, 2001. 50; 609-613).

GLP-1 receptor agonists (such as exenatide) may improve blood glucose control in T2DM patients by reducing fasting and postprandial glucose, and their main pharmacological activities include: (i) an increase in glucose-dependent insulin secretion, (ii) a glucagon inhibitory activity in hyperglycemic conditions, and (iii) a delay in gastric emptying rate, resulting in delayed absorption of glucose from dietary sources (referring to Holst (Physiol. Rev. 2007, 87, 1409) and Meier (Nat. Rev. Endocrinol. 2012, 8728)).

Although these receptor agonists provide better therapeutic effects for patients, they still only account for a limited proportion of diabetes prescriptions. One of the important reasons is that they are all macromolecular peptide-based medicaments, which need to be administered by invasive subcutaneous injection. Compared with oral administration, the patient compliance is poor. PF-06882961 is an oral micromolecular GLP-1 receptor agonist developed by Pfizer. The first-phase clinical results of PF-06882961 show that it has significant effects on reducing blood glucose and weight in patients with Type-2 diabetes mellitus.

Although the oral micromolecular GLP-1 receptor agonist PF-06882961 has already been reported currently, there is still a need to develop new oral micromolecular compounds with better pharmacodynamic and pharmacokinetic properties for prevention and/or treatment of GLP-1 activity-related diseases.

Information disclosed in the portion of the background art is only intended to enhance the overall background understanding of the present application, and should not be regarded as acknowledging or implying in any forms that the information constitutes the prior art already known to those skilled in the art.

### Summary of the Invention

The purpose of the present application is to provide compounds as GLP-1 receptor agonists and uses thereof. The compounds have excellent GLP-1R receptor agonist activity and significant blood glucose-reducing effect, have better pharmacokinetic characteristics, and provide more options for the prevention and/or treatment of GLP-1 activity-related diseases, conditions, or disorders.

Specifically, in a first aspect, the present application provides a compound as shown in General formula (I) or a pharmaceutically acceptable salt thereof: 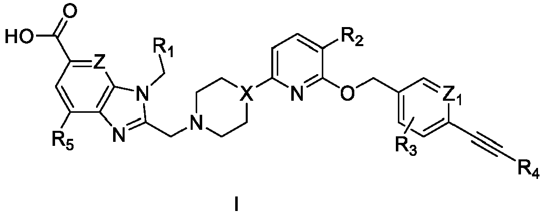 wherein,
R₁ is selected from (S)-oxacyclobutan-2-yl, 1-tetrahydrofuran-3-yl, 1-ethyl-1*H*-imidazol-4-yl, or oxazol-2-yl;
R₂ is selected from hydrogen or halogen;
R₃ is selected from hydrogen, C₁-C₆ alkyl or halogen;
R₄ is selected from hydrogen, optionally substituted C₁-C₆ alkyl, or optionally substituted C₃-C₇ cycloalkyl; when R₄ is substituted C₁-C₆ alkyl or substituted C₃-C₇ cycloalkyl, a substituent may be at any available linkage point, and preferably the substituent is independently selected from one or more of the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group;
R₅ is selected from hydrogen or halogen; and
X, Z and Z₁ are each independently selected from -CH or nitrogen.

As a preferred embodiment, in the compound shown in Formula (I) above,
R₁ is (*S*)-oxacyclobutan-2-yl;
R₂ is selected from hydrogen or halogen;
R₃ is selected from hydrogen, C₁-C₃ alkyl or halogen;
R₄ is selected from hydrogen, optionally substituted C₁-C₆ alkyl, or optionally substituted C₃-C₇ cycloalkyl; when R₄ is substituted C₁-C₆ alkyl or substituted C₃-C₇ cycloalkyl, a substituent may be at any available linkage point, and preferably the substituent is independently selected from one or more of the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group;
R₅ is selected from hydrogen or fluorine; and
X, Z and Z₁ are each independently selected from -CH or nitrogen.

In a preferred embodiment of the compound shown in Formula (I) above, R₁ is (S)-oxacyclobutan-2-yl; R₂ is selected from hydrogen or fluorine; R₃ is selected from hydrogen, methyl, fluorine, or chlorine; R₄ is selected from hydrogen, hydroxymethyl, or cyclopropyl; R₅ is selected from hydrogen or fluorine; X and Z are each independently selected from -CH or nitrogen; and Z₁ is -CH. As a further preferred embodiment, the compound shown in Formula (I) above or a pharmaceutically acceptable salt thereof is selected from:

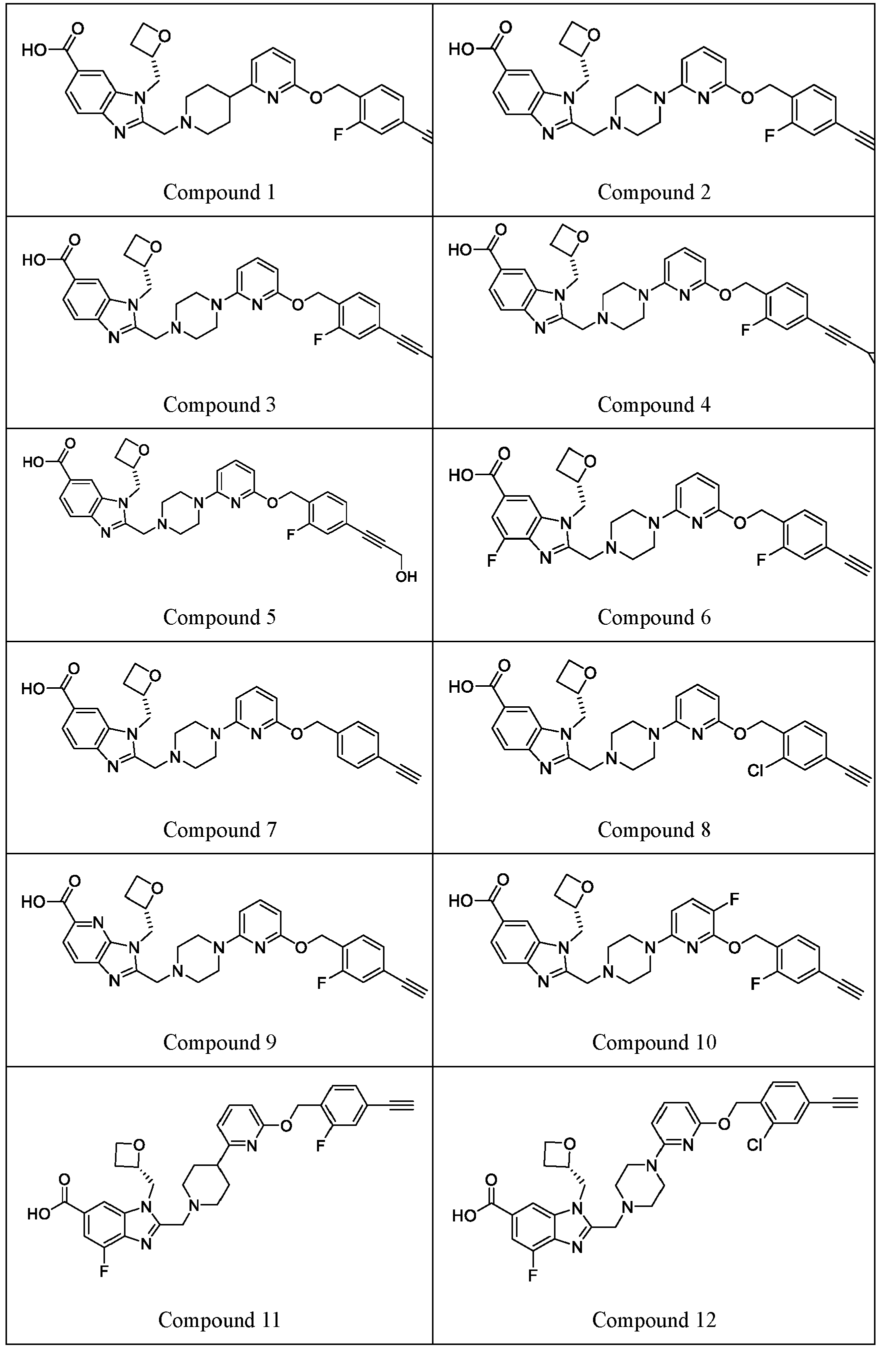

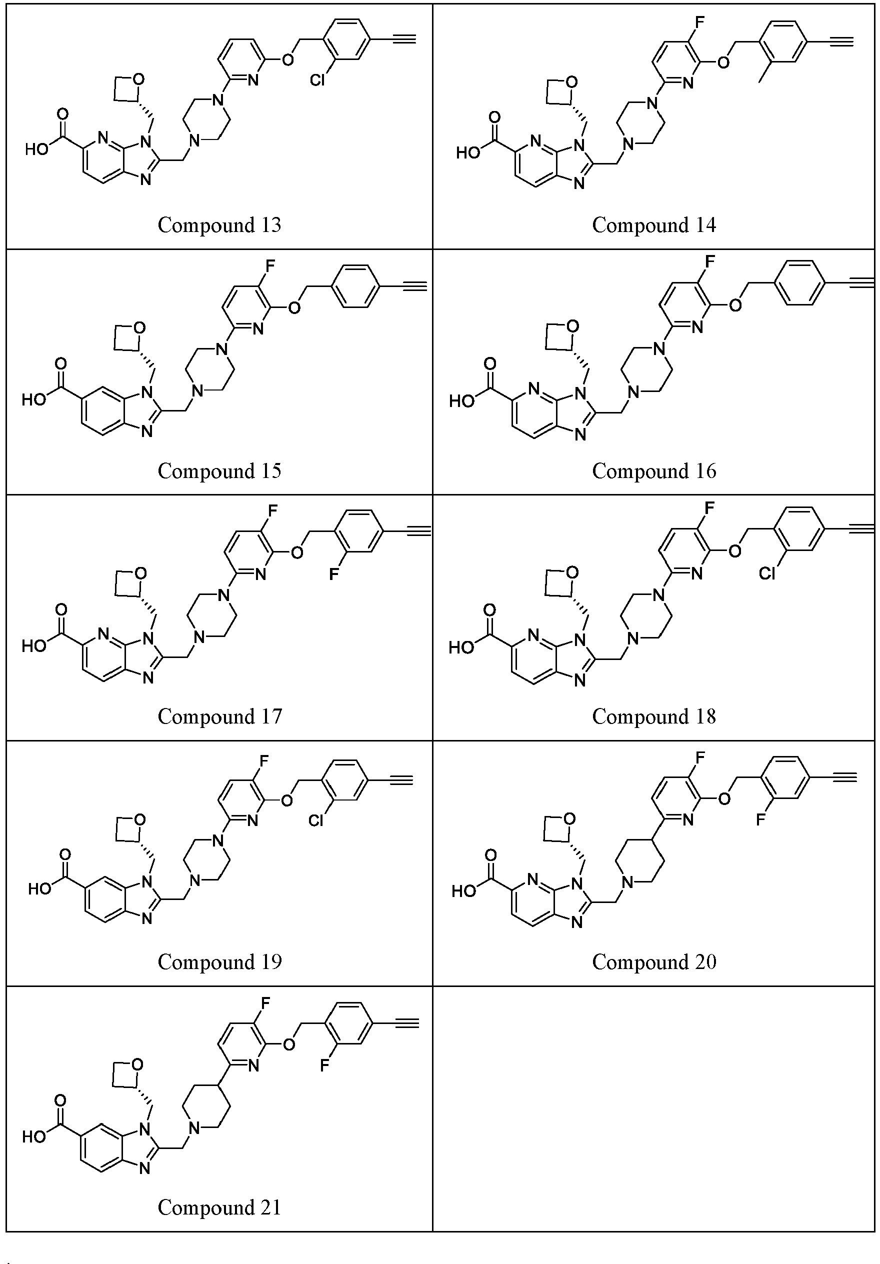

In a second aspect, the present application provides a pharmaceutical composition comprising the compound or a pharmaceutically acceptable salt thereof as described in the above first aspect and a pharmaceutically acceptable carrier, diluent, and/or excipient.

In a third aspect, the present application provides the use of the compound or a pharmaceutically acceptable salt thereof as described in the above first aspect in the preparation of a glucagon-like peptide-1 receptor agonist.

In a fourth aspect, the present application provides the use of the compound or a pharmaceutically acceptable salt thereof as described in the above first aspect or the pharmaceutical composition as described in the above second aspect in the preparation of a medicament for the prevention and/or treatment of glucagon-like peptide-1 activity-related diseases, conditions, or disorders, wherein the diseases, conditions, or disorders may be regulated or treated by activating a glucagon-like peptide-1 receptor.

In a preferred embodiment, the diseases, conditions, or disorders are selected from the group consisting of the followings:
type 1 diabetes, type 2 diabetes mellitus, prediabetes, idiopathic type 1 diabetes mellitus, latent autoimmune diabetes in adults (LADA), early-onset type 2 diabetes (EOD), young-onset atypical diabetes (YOAD), maturity-onset diabetes of the young (MODY), malnutrition-related diabetes mellitus, gestational diabetes mellitus, hyperglycemia, insulin resistance, glucose intolerance, diabetic neuropathy, diabetic nephropathy, kidney disease, diabetic retinopathy, adipocyte dysfunction, visceral adipocyte accumulation, sleep apnoea, obesity, eating disorder, weight gain caused by the use of other medicaments, sugar fanatic, dyslipidemia, hyperinsulinemia, non-alcoholic fatty liver disease (NAFLD), non-alcoholic steatohepatitis (NASH), fibrosis, sclerosis, hepatocellular carcinoma, cardiovascular disease, atherosclerosis, coronary artery disease, peripheral vascular disease, hypertension, endothelial dysfunction, injured vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipidemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome X, premenstrual syndrome, angina pectoris, thrombosis, atherosclerosis, transient ischemic attack, vascular restenosis, poor glucose metabolism, impaired fasting glucose condition, hyperuricemia, gout, erectile dysfunction, skin and connective tissue abnormalities, psoriasis, foot ulcer, ulcerative colitis, hyper-Apo B lipoproteinemia, Alzheimer's disease, schizophrenia, cognitive impairment, inflammatory bowel disease, short bowel syndrome, Crohn's disease, colitis, irritable bowel syndrome, and polycystic ovary syndrome.

Further preferably, the diseases are type 2 diabetes mellitus or the following conditions related thereto: hyperglycemia, insulin resistance, glucose intolerance, diabetic neuropathy, diabetic nephropathy, diabetic retinopathy, obesity, dyslipidemia, hypertension, hyperinsulinemia or non-alcoholic fatty liver disease (NAFLD).

In a fifth aspect, the present application further provides a method for activating the glucagon-like peptide-1 receptor, and it includes the following steps: administering an effective amount of the compound or a pharmaceutically acceptable salt thereof as described in the above first aspect or the pharmaceutical composition as described in the above second aspect to a subject who needs to activate the glucagon-like peptide-1 receptor.

In a sixth aspect, the present application further provides a method for preventing and/or treating diseases, conditions, or disorders related to glucagon-like peptide-1 activity, and it includes: administering a prophylactic and/or therapeutic effective amount of the compound or a pharmaceutically acceptable salt thereof as described in the above first aspect or the pharmaceutical composition as described in the above second aspect to a subject in need.

Specifically, the diseases, conditions, or disorders may be regulated or treated by activating the glucagon-like peptide-1 receptor.

In a preferred embodiment, the diseases, conditions, or disorders are selected from the group consisting of the followings:
type 1 diabetes, type 2 diabetes mellitus, prediabetes, idiopathic type 1 diabetes mellitus, latent autoimmune diabetes in adults (LADA), early-onset type 2 diabetes (EOD), young-onset atypical diabetes (YOAD), maturity-onset diabetes of the young (MODY), malnutrition-related diabetes mellitus, gestational diabetes mellitus, hyperglycemia, insulin resistance, glucose intolerance, diabetic neuropathy, diabetic nephropathy, kidney disease, diabetic retinopathy, adipocyte dysfunction, visceral adipocyte accumulation, sleep apnoea, obesity, eating disorder, weight gain caused by the use of other medicaments, sugar fanatic, dyslipidemia, hyperinsulinemia, non-alcoholic fatty liver disease (NAFLD), non-alcoholic steatohepatitis (NASH), fibrosis, sclerosis, hepatocellular carcinoma, cardiovascular disease, atherosclerosis, coronary artery disease, peripheral vascular disease, hypertension, endothelial dysfunction, injured vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipidemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome X, premenstrual syndrome, angina pectoris, thrombosis, atherosclerosis, transient ischemic attack, vascular restenosis, poor glucose metabolism, impaired fasting glucose condition, hyperuricemia, gout, erectile dysfunction, skin and connective tissue abnormalities, psoriasis, foot ulcer, ulcerative colitis, hyper-Apo B lipoproteinemia, Alzheimer's disease, schizophrenia, cognitive impairment, inflammatory bowel disease, short bowel syndrome, Crohn's disease, colitis, irritable bowel syndrome, and polycystic ovary syndrome.

Further preferably, the diseases are type 2 diabetes mellitus or the following conditions related thereto: hyperglycemia, insulin resistance, glucose intolerance, diabetic neuropathy, diabetic nephropathy, diabetic retinopathy, obesity, dyslipidemia, hypertension, hyperinsulinemia or non-alcoholic fatty liver disease (NAFLD).

### Beneficial Effect

The compounds shown in Formula (I) or a pharmaceutically acceptable salt thereof provided in the present application have excellent GLP-1R receptor agonist activity and significant blood glucose-reducing effect, have better pharmacokinetic characteristics, and provide more options for the prevention and/or treatment of GLP-1 activity-related diseases, conditions, or disorders, and thereby have better clinical application prospects.

### Brief Description of the Drawings

One or more examples are exemplarily described by corresponding pictures in drawings, and these exemplary descriptions do not constitute limitations on the examples. The specialized word "exemplarily/exemplary" here means "used as an example, embodiment, or description". Any examples described here as the "exemplary" descriptions do not need to be explained as superior to or better than other examples.

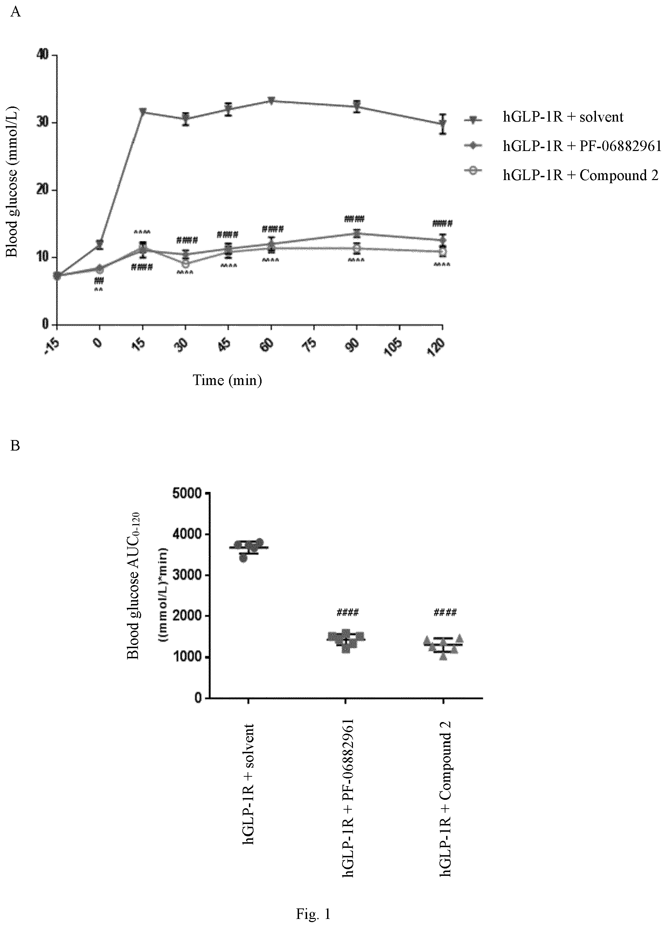

Fig. 1 shows experimental results of a mouse intraperitoneal glucose tolerance test (IPGTT) of Compound 2 recorded in Example 24 of the present application; herein, Fig. A shows that: compared to a known control medicament PF-06882961, Compound 2 of the present application has an effect on blood glucose levels in hGLP-1R IPGTT, herein the horizontal coordinate shows a treatment time (unit: min), the vertical coordinate shows a blood glucose level (unit: mmol/L), and "#" represents the significance of a "hGLP-1R + PF-06882961" group compared to a "hGLP-1R + solvent" group, herein "##" represents P<0.01, and "####" represents P<0.0001; "^" represents the significance of a "hGLP-1R + Compound 2" group compared to the "hGLP-1R + solvent" group, herein "^^" represents P<0.01, and "^^^^" represents P<0.0001; and Fig. B shows that compared to the known control medicament PF-06882961, Compound 2 of the present application shows an area under curve (AUC) of blood glucose level changes after glucose administration in IPGTT, herein the horizontal coordinate shows a group, and the vertical coordinate shows a blood glucose AUC₀₋₁₂₀ (unit: (mmol/L)*min), "####" represents the significance of the "hGLP-1R + PF-06882961" group and the "hGLP-1R + Compound 2" group compared to the "hGLP-1R + solvent" group, and P<0.0001.

### Detailed Description of the Invention

In order to make the purposes, technical solutions, and advantages of the present application clearer, the technical solutions in the examples of the present application are clearly and completely described below. Apparently, the examples described are a part of the examples of the present application, not all of the examples. Based on the examples in the present application, all other examples obtained by those of ordinary skill in the art without creative labor shall fall within the scope of protection of the present application.

In addition, in order to describe the present application better, numerous specific details are provided in the following specific implementation modes. It should be understood by those skilled in the art that, without certain specific details, the present application may also be implemented. In some examples, raw materials, methods and the like familiar to those skilled in the art are not described in detail, as to highlight the main idea of the present application.

Unless otherwise explicitly stated, throughout the entire description and claims, the term "include(s)/including" or its variations such as "contain(s)/containing" or "comprise(s)/comprising" may be understood to include the stated constituent parts, without excluding other constituent parts.

### Definition of Term

It should be noted that, unless otherwise stated, terms used in the description and claims have the following meanings.

The term "C₁-C₆ alkyl" refers to an alkyl containing 1 to 6 carbon atoms, preferably an alkyl containing 1 to 4 carbon atoms, such as methyl. Non-restrictive examples include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl 1,3-dimethylbutyl, 2-ethylbutyl, 2-methylpentyl, 3-methylpentyl, 4-methylpentyl, 2,3-dimethylbutyl and the like. The alkyl may be substituted or unsubstituted, and when substituted, a substituent may be at any available linkage point, and preferably the substituent is independently selected from one or more of the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "C₃-C₇ cycloalkyl" refers to a saturated or partially unsaturated monocyclic or polycyclic cyclic hydrocarbon substituent containing 3 to 7 carbon atoms. Non-restrictive examples of monocyclic cycloalkyl include cyclopropyl, cyclobutyl, cyclopentyl, cyclopentenyl, cyclohexyl, cyclohexenyl, cyclohexadienyl, cycloheptyl, cycloheptrienyl and the like, and preferably cyclopropyl and cyclopentyl. Polycyclic cycloalkyl includes cycloalkyl of a spiro ring, a fused ring, and a bridged ring. The cycloalkyl may be optionally substituted or unsubstituted, and when substituted, the substituent is preferably independently selected from one or more of the following groups: alkyl, alkenyl, alkynyl, alkoxy, alkylthio, alkylamino, halogen, mercapto, hydroxyl, nitro, cyano, cycloalkyl, heterocyclic alkyl, aryl, heteroaryl, cycloalkoxy, heterocyclic alkoxy, cycloalkylthio, heterocyclic alkylthio, oxo, amino, haloalkyl, hydroxyalkyl, carboxyl or carboxylic acid ester group.

The term "halogen" refers to fluorine, chlorine, bromine, or iodine, and preferably fluorine or chlorine.

The term "optional" or "optionally" means that an event or environment described subsequently can but does not necessarily occur, and this description includes cases in which the event or environment occurs or does not occur. For example, a "heterocyclic alkyl group optionally substituted by an alkyl" means that the alkyl may but does not necessarily exist, and this description includes a situation that the heterocyclic alkyl group is substituted by the alkyl and a situation that the heterocyclic alkyl group is not substituted by the alkyl.

"Substituted" means that one or more hydrogen atoms in a group, preferably at most 5, and more preferably 1-3 hydrogen atoms, are independently substituted by a corresponding number of substituents. It is apparent that the substituents are only in their possible chemical positions, and those skilled in the art may determine (by experiments or theories) possible or impossible substitutions without excessive effort. For example, an amino or hydroxyl with a free hydrogen may be unstable when linked with carbon atoms with an unsaturated (such as ene) bond.

### Pharmaceutically Acceptable Salt of Compound in Formula (I) and Preparation Method Thereof

It should be noted that the pharmaceutically acceptable salt of the compound in Formula (I) in the present application includes an acid addition salt and an alkali salt.

The suitable acid addition salt is formed by acids that form non-toxic salts. Examples of the acid addition salt include acetate, adipate, aspartate, benzoate, benzenesulfonate, bicarbonate/carbonate, bisulfate/sulfate, borate, camphor sulfonate, citrate, cyclohexylamine sulfonate, ethanedisulfonate, ethanesulfonate, formate, fumarate, gluceptate, gluconate, glucuronate, hexafluorophosphate, hibenzate, hydrochloride/chloride, hydrobromate/bromide, hydroiodate/iodide, hydroxyethyl sulfonate, lactate, malate, maleate, malonate, methanesulfonate, methylsulfate, naphthalate, 2-naphthalene sulfonate, nicotinate, nitrate, orotate, oxalate, palmitate, pamoate, phosphate/hydrophosphate/dihydrogen phosphate, pyroglutamate, saccharate, stearate, succinate, tannate, tartrate, tosylate, trifluoroacetate, 1,5-naphthalene disulfonate, and xinafoate.

The suitable alkali salt is formed by alkalis that form non-toxic salts. Examples of the alkali salt include aluminum salt, arginine salt, benzathine salt, calcium salt, choline salt, diethylamine salt, bis(2-hydroxyethyl)amine(diolamine) salt, glycine salt, lysine salt, magnesium salt, meglumine salt, 2-aminoethanol(olamine) salt, potassium salt, sodium salt, 2-amino-2-(hydroxymethyl)prop-1,3-diol(tris or tromethamine) salt, and zinc salt.

The compound in Formula (I) of the present application may also form semi-salts of acids and alkalis, such as semi-sulfate and semi-calcium salt. For a review of suitable salts, please refer to Handbook of Pharmaceutical Salts: Properties, Selection, and Use by Stahl and Wermuth (Wiley-VCH, 2002).

The pharmaceutically acceptable salt of the compound in Formula (I) of the present application may be prepared by one or more of the following three methods:
(i) the compound in Formula (I) is reacted with a desired acid or alkali;
(ii) a desired acid or alkali is used, and an acid or alkali unstable protecting group is removed from an appropriate precursor of the compound in Formula (I), or an appropriate cyclic precursor (such as lactone or lactam) is ring-opened; and
(iii) one salt of the compound in Formula (I) is reacted with an appropriate acid or alkali or converted into another salt with the help of an appropriate ion exchange column.

### Preparation of Compound in Formula (I)

The compound in Formula (I) provided in the present application may be prepared by using common knowledge of those skilled in the synthetic organic chemistry field and the general and specific methods described below. This type of common knowledge may be found in standard reference books such as Comprehensive Organic Chemistry, Ed .Barton and Ollis, Elsevier; Com prehensive Organic Transformations: A Guide to Functional Group Preparations ,Larock John Wiley and Sons; and Compendium of Organic Synthetic Methods, Vol .I-XII (issued by Wiley-Interscience). Raw materials used in the present application are available in the market or may be prepared by using conventional methods known in this technology.

Some specific preparation examples and properties and effect experiments of the compound in Formula (I) of the present application are provided below.

### Example 1: Synthesis of Compound 1

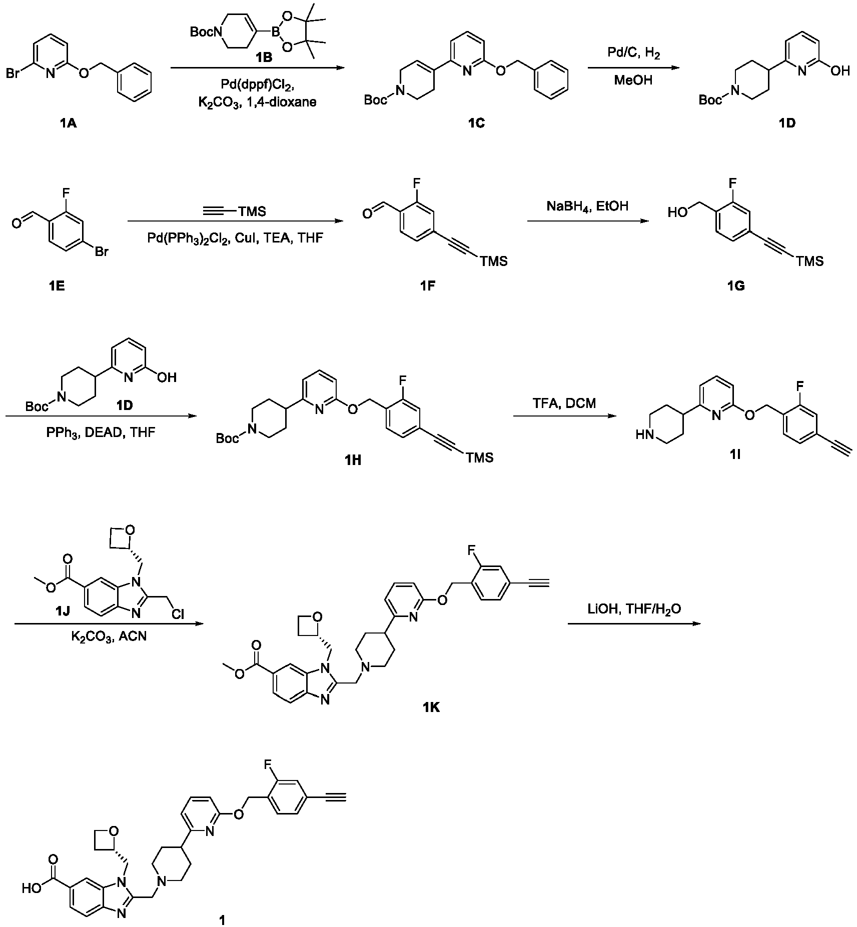

### Specific steps were as follows:

### Step 1-1: Synthesis of Compound 1C

Compound **1A** (5 g, 18.9 mmol), Compound **1B** (7.0 g, 22.6 mmol), Pd(dppf)Clz (0.5 g, 0.68 mmol), and potassium carbonate (5.2 g, 37.6 mmol) were successively added into a 100 mL microwave tube, then a mixed solvent of 1,4-dioxane (50 mL) and water (5 mL) was added thereinto. The obtained mixture was microwave-heated to 120 °C under an argon atmosphere and reacted for 3 h. After the reaction solution was concentrated, it was directly separated by a silica gel column, and eluted with 0-20% ethyl acetate (EA)/petroleum ether (PE). A corresponding solution was collected and concentrated to obtain a colorless oily substance, namely Compound **1C** (6.0 g). LCMS (ESI +): 367 (M + H)⁺.

### Step 1-2: Synthesis of Compound 1D

Compound **1C** (6 g, 16.3 mmol) obtained in Step **1-1** was dissolved in methanol (60 mL) in a 150 mL round-bottom flask, and Pd/C (10%, 50% moisture) (0.6 g) was added thereinto. After being replaced with hydrogen for 3 times, the mixture was continuously stirred for 16 h, and the reaction completion was detected by thin layer chromatography (TLC). The obtained reaction solution was filtered, mother solution was concentrated, and a mixed solution (50 mL, 5:1, v:v) of petroleum ether and ethyl acetate was added and beaten for 2 h. Filtration was conducted to precipitate a white solid, namely Compound **1D** (2.0 g). LCMS (ESI +): 279 (M + H)⁺.

### Step 1-3: Synthesis of Compound 1F

Compound **1E** (1.42 g, 7 mmol, purchased from Bide Pharm, cat. #: BD10249), Pd(PPh₃)₂Cl₂ (98 mg, 0.14 mmol), and PPh₃ (28 mg, 0.105 mmol) were successively added into a 50 mL round-bottom flask, and tetrahydrofuran (THF) (22 mL) was finally added thereinto. The obtained mixture was replaced with nitrogen for 3 times and stirred at room temperature (25 °C) for 20 min, triethylamine (1.89 mL, 13.65 mmol) and trimethylsilyacetylene (1.09 mL, 7.7 mmol) were added thereinto and stirred for 20 min, then copper iodide (40 mg, 0.21 mmol) was added thereinto and stirred at room temperature for 5 h, and the reaction completion was detected by TLC. The reaction solution was concentrated to obtain a residue which was a brown solid, then n-pentane (50 mL) was added thereinto. The mixture was filtered after being stirred for 10 min, and mother solution was concentrated to obtain a yellow solid, namely Compound **1F** (1.45 g), which was directly used for the next step.

### Step 1-4: Synthesis of Compound 1G

Compound **1F** (1.45 g, 6.6 mmol) obtained in Step **1-3** was added into a 50 mL round-bottom flask, and ethanol (25 mL) was added thereinto to obtain a light yellow solution, which was cooled to 0-5 °C under stirring. Sodium borohydride (0.25 g, 6.6 mmol) was added into the solution in batches, and there were bubbles emerged. It was continuously stirred for 30 min, and the reaction completion was detected by TLC. Saturated ammonium chloride solution (50 mL) was dropwise added, after being stirred for 10 min, dichloromethane (DCM) was added for extraction and solution separation, and a water phase was extracted twice with dichloromethane. Organic phases were merged, dried with magnesium sulfate, filtered, concentrated, separated, purified with a silica gel column, and eluted (0-50% DCM/PE). A corresponding solution was collected and concentrated to obtain a colorless oily substance, namely Compound **1G** (1.3 g).

### Step 1-5: Synthesis of Compound 1H

Compound **1D** (0.28 g, 1 mmol) and Compound **1G** (0.22 g, 1 mmol) obtained according to the above steps, and PPh₃ (0.39 g, 1.5 mmol) were successively added into a 50 mL round-bottom flask, and anhydrous tetrahydrofuran (15 mL) was added thereinto to obtain a mixed solution. After being replaced with nitrogen for 3 times, the solution was cooled to 0-5 °C under stirring, then diethyl azodicarboxylate (DEAD) (0.26 g, 1.5 mmol) was dropwise added carefully, and the temperature was controlled to be below 5 °C. The mixture was continuously stirred for 16 h after being added, and the reaction completion was detected by TLC. After the reaction solution was concentrated, it was directly separated by a silica gel column, and eluted with 0-20% EA/PE. A corresponding solution was collected and concentrated to obtain a colorless oily substance, namely Compound **1H** (0.20 g). LCMS (ESI +): 427 (M + H)⁺.

### Step 1-6: Synthesis of Compound 11

Compound **1H** (0.20 g, 0.41 mmol) obtained in Step **1-5** was dissolved in dichloromethane (10 mL) in a 25 mL round-bottom flask, trifluoroacetic acid (2 mL) was added under stirring, and there were bubbles emerged. The mixture was directly concentrated to dry after being continuously stirred for 16 h, then a sodium bicarbonate solution was added, and pH was adjusted to 8-9. The obtained mixture was extracted with dichloromethane for 3 times, and organic phases were merged, filtered and concentrated after being dried with anhydrous magnesium sulfate, to obtain an oily substance, namely Compound **1I** (0.11 g), which was directly used for the next step. LCMS (ESI +): 298 (M + H)+.

### Step 1-7: Synthesis of Compound 1K

Compound **1I** (0.10 g, 0.34 mmol, prepared according to Step **1-6**) was dissolved in acetonitrile (10 mL) in a 25 mL round-bottom flask, and potassium carbonate (0.09 g, 0.67 mmol) was added thereinto under stirring. After 10 min, Compound **1J** (0.09 g, 0.34 mmol) was added, and the mixture was heated to 45-50 °C and reacted for 3 h. The reaction completion was detected by TLC. After the reaction solution was directly concentrated to dry, column chromatography was performed and the solution was eluted with 0-5% MeOH/DCM, and a corresponding solution was collected and concentrated, to obtain a colorless oily substance, namely Compound **1K** (0.10 g). LCMS (ESI +): 569 (M + H)⁺.

### Step 1-8: Synthesis of Compound 1

Compound **1K** (0.10 g, 0.17 mmol) obtained in Step **1-7** was dissolved in tetrahydrofuran (5 mL) and water (1.5 mL) in a 25 mL round-bottom flask, and lithium hydroxide (5 mg, 0.21 mmol) was added under stirring. It was reacted at room temperature for 16 h. Acetic acid was added to adjust pH to 5-6, and the mixture was stirred for 5 min, and extracted with dichloromethane for 3 times. Organic phases were merged, dried with anhydrous magnesium sulfate, filtered, and concentrated to obtain a colorless oily substance, namely Compound **1** (30 mg). LCMS (ESI +): 555 (M + H)⁺.

### Example 2: Synthesis of Compound 2

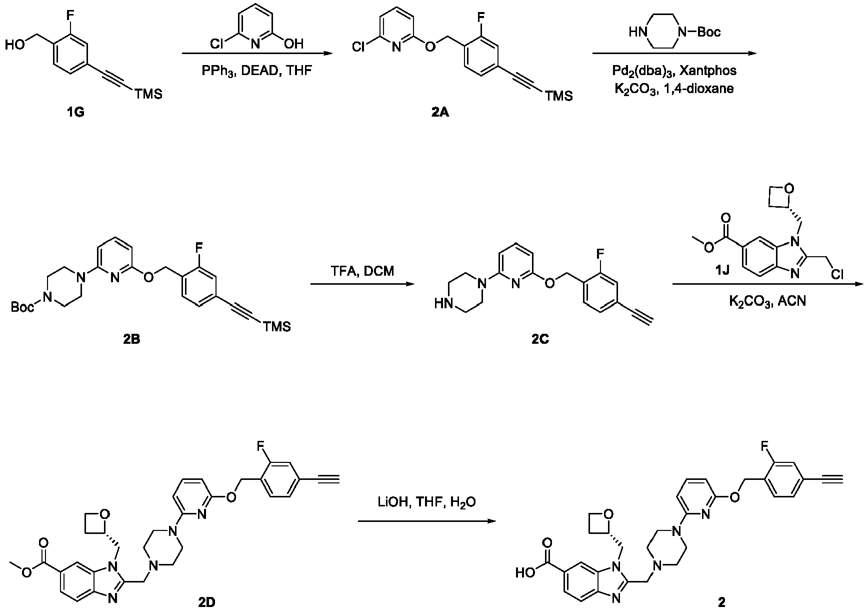

### Specific steps were as follows:

### Step 2-1: Synthesis of Compound 2A

Compound **1G** (0.3 g, 1.35 mmol, prepared according to Step **1-4** in Example **1**), 2-chloro-6-hydroxypyridine (0.19 g, 1.47 mmol), and PPh₃ (0.5 g, 1.90 mmol) were successively added into a 25 mL round-bottom flask, and anhydrous tetrahydrofuran (10 mL) was added to obtain a solution. The solution was replaced with nitrogen for 3 times, and cooled to 0-5 °C under stirring. DEAD (0.35 g, 1.90 mmol) was dropwise added carefully, the temperature was controlled to be below 5 °C, and the mixture was continuously stirred for 16 h after the addition was completed. The reaction completion was detected by TLC. After the reaction solution was concentrated to dry, silica gel column chromatography was directly performed and the solution was eluted with 0-20% EA/PE. A corresponding solution was collected and concentrated to obtain a colorless oily substance, namely Compound **2A** (0.25 g). LCMS (ESI +): 334 (M + H)⁺.

### Step 2-2: Synthesis of Compound 2B

Compound **2A** (0.20 g, 1.35 mmol) prepared according to Step **2-1,** N-Boc piperazine (0.13 g, 1.47 mmol), Pd₂(dba)₃ (30 mg), Xantphos (37 mg, 1.90 mmol), and potassium carbonate (0 13 g) were successively added into a 10 mL microwave reaction tube, and 1,4-dioxane (5 mL) was added thereinto. The mixture was covered after being bubbled with argon for 30 s, and microwave-reacted at 110 °C for 2 h. The reaction completion was detected by TLC. After the reaction solution was concentrated to dry, it was directly separated and purified by a silica gel column and eluted with 0-30% EA/PE. A corresponding solution was collected, and concentrated to obtain a colorless oily substance, namely Compound **2B** (0.16 g). LCMS (ESI +): 484 (M + H)⁺.

### Step 2-3: Synthesis of Compound 2C

Compound **2B** (160 mg, 0.33 mmol) prepared in Step **2-2** was dissolved in dichloromethane (5 mL) in a 25 mL round-bottom flask, trifluoroacetic acid (2 mL) was added under stirring, and there were bubbles emerged. The mixture was continuously stirred at room temperature for 6 h, and the reaction completion was detected by TLC. The reaction solution was concentrated to obtain Compound 2C (80 mg), which was directly used for the next step of the reaction. LCMS (ESI +): 312 (M + H)⁺.

### Step 2-4: Synthesis of Compound 2D

Compound **2C** (80 mg, 0.26 mmol) prepared in Step **2-3** was dissolved in acetonitrile (5 mL) in a 10 mL round-bottom flask, and potassium carbonate (100 mg, 0.72 mmol) was added under stirring. The mixture was continuously stirred at room temperature for 10 min, and Compound 1J (80 mg, 0.27 mmol) was added thereinto. The obtained mixture was heated to 50-55 °C and reacted for 2 h, and the reaction completion was detected by TLC. After the reaction solution was concentrated to dry, it was directly purified by a preparation plate and expanded with 5% MeOH/DCM. It was eluted after corresponding fluorescent silica gel was collected, and concentrated to obtain a colorless oily substance, namely Compound **2D** (40 mg). LCMS (ESI +): 570 (M + H)⁺.

### Step 2-5: Synthesis of Compound 2

Compound **2D** (40 mg, 0.07 mmol) prepared in Step **2-4** was dissolved in tetrahydrofuran (5 mL) and water (1 mL) in a 10 mL round-bottom flask, and lithium hydroxide (2.4 mg, 0.1 mmol) was added under stirring. The mixture was reacted at room temperature for 16 h, and the reaction completion was detected by TLC. 3 drops of acetic acid was added and stirred for 5 min, the obtained solution was directly purified by a preparation plate after being concentrated to dry, and expanded with 10% MeOH/DCM. It was eluted after corresponding fluorescent silica gel was collected, and concentrated to obtain a white solid, namely Compound 2 (35 mg). LCMS (ESI +): 556 (M + H)⁺.

### Example 3: Synthesis of Compound 3

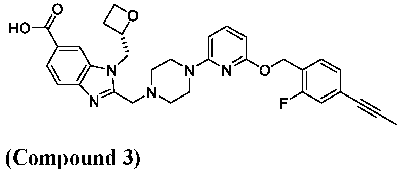

According to the synthesis route of Example 1, in Step **1-3,** trimethylsilyacetylene was replaced with propyne, to obtain a compound (4-propargyl-2-fluorophenyl)methanol; and according to the synthesis route of Example 2, in Step **2-1,** Compound **1G** was replaced with the compound (4-propargyl-2-fluorophenyl)methanol, to obtain Compound 3. LCMS (ESI +): 570.43 (M + H)+.

### Example 4: Synthesis of Compound 4

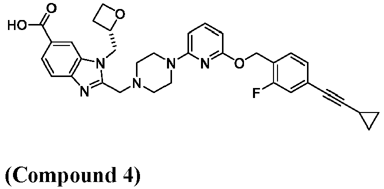

According to the synthesis route of Example 1, in Step **1-3,** trimethylsilyacetylene was replaced with cyclopropyl acetylene, to obtain a compound (4-cyclopropylacetenyl-2-fluorophenyl)methanol; and according to the synthesis route of Example 2, in Step **2-1,** Compound **1G** was replaced with the compound (4-cyclopropylacetenyl-2-fluorophenyl)methanol, to obtain Compound 4. LCMS (ESI +): 596.35 (M + H)+.

### Example 5: Synthesis of Compound 5

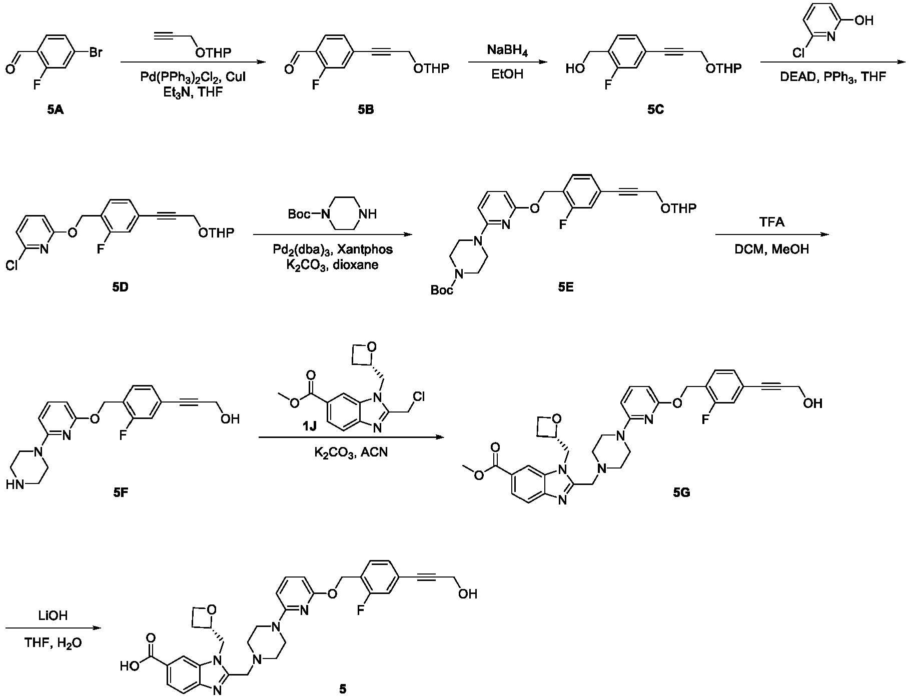

### Specific steps were as follows:

### Step 5-1: Synthesis of Compound 5E

According to the synthesis route of Example **1,** in Step **1-3,** trimethylsilyacetylene was replaced with 2-(2-propargyloxy)tetrahydropyran, to obtain Compound **5C;** and according to the synthesis route of Example **2,** in Step **2-1,** Compound **1G** was replaced with Compound **5C,** to obtain Compound **5E.** LCMS (ESI +): 526.47 (M + H)+.

### Step 5-2: Synthesis of Compound 5F

Compound **5E** (120 mg, 0.23 mmol) obtained in Step **5-1** was added into a mixed solvent of dichloromethane (6 mL) and methanol (0.5 mL), and then trifluoroacetic acid (2 mL) was added. The reaction solution was stirred at room temperature for about 2 h. The reaction solution was concentrated under a reduced pressure to obtain a light yellow oily compound **5F.** The crude product was not further purified and directly used for the next step of the reaction. LCMS (ESI +): 342.06 (M + H)+.

### Step 5-3: Synthesis of Compound 5

According to the synthesis route of Example **2,** in Step **2-4,** Compound **2C** was replaced with Compound **5F,** to obtain Compound **5.** LCMS (ESI +): 586.37 (M + H)+.

### Example 6: Synthesis of Compound 6

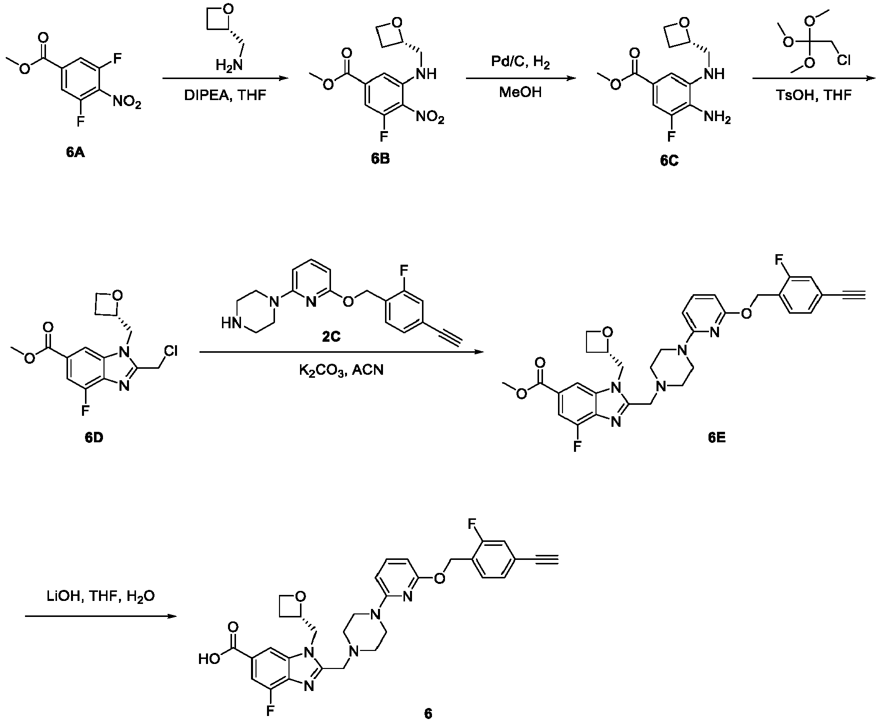

### Specific steps were as follows:

### Step 6-1: Synthesis of Compound 6B

Compound **6A** (10.8 g, 0.05 mol) was added to tetrahydrofuran (100 mL) and stirred to dissolve, then N,N-diisopropylethylamine (32.3 g, 0.25 mol) was added, and the obtained mixture was cooled to 0 °C. (S)-oxacyclobutane-2-methylamine (4.7g, 0.053mol) was dropwise added, the temperature was slowly raised to 25 °C after the addition was completed. After the mixture was stirred for 1 h, ethyl acetate (100 mL) was added. The organic phase was dried by anhydrous sodium sulfate after being washed with saturated salt water for three times and filtered, and mother solution was concentrated to obtain a yellow solid, namely Compound **6B** (13.5g), which was directly used for the next step.

### Step 6-2: Synthesis of Compound 6C

Compound **6B** (13.5 g, 0.047 mol) was added into methanol (150 mL) and stirred to dissolve, then 10% Pd/C (3.5 g, 50% water) was added. The mixture was replaced with hydrogen for 3 times under stirring, and filtered after being stirred at room temperature for 18 h. A filter cake was washed twice with methanol and organic phases were merged and concentrated to obtain a dark oily substance, namely Compound **6C** (11 g), which was directly used for the next step.

### Step 6-3: Synthesis of Compound 6D

Compound **6C** (11 g, 0.043 mol) was added to tetrahydrofuran (100 mL) and stirred to dissolve, then 2-chloro-1,1,1-trimethoxyethane (10 g, 0.064 mol) andp-toluenesulfonic acid (0.83 g, 0.005 mol) were added. The obtained mixture was heated to 60 °C and reacted for 2 h. After the reaction solution was cooled to room temperature and directly concentrated to dry, column chromatography was performed and the solution was eluted with 0-5% MeOH/DCM, to obtain an oily substance, namely Compound **6D** (8 g), which was directly used for the next step. LCMS (ESI +): 313.1 (M + H)+.

### Step 6-4: Synthesis of Compound 6

According to the synthesis route of Example **2,** in Step **2-4,** Compound **1J** was replaced with Compound **6D,** to obtain Compound 6. **LCMS (ESI +): 573.4 (M + H)+.**

### Example 7: Synthesis of Compound 7

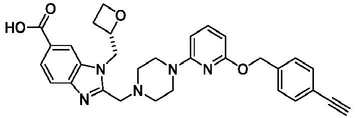

### (Compound 7)

According to the synthesis route of Example **2,** in Step **2-1,** Compound **1G** was replaced with a compound 4-(trimethylsilylacetenyl)benzyl methanol, to obtain Compound 7. LCMS (ESI +): 536.4 (M + H)+.

### Example 8: Synthesis of Compound 8

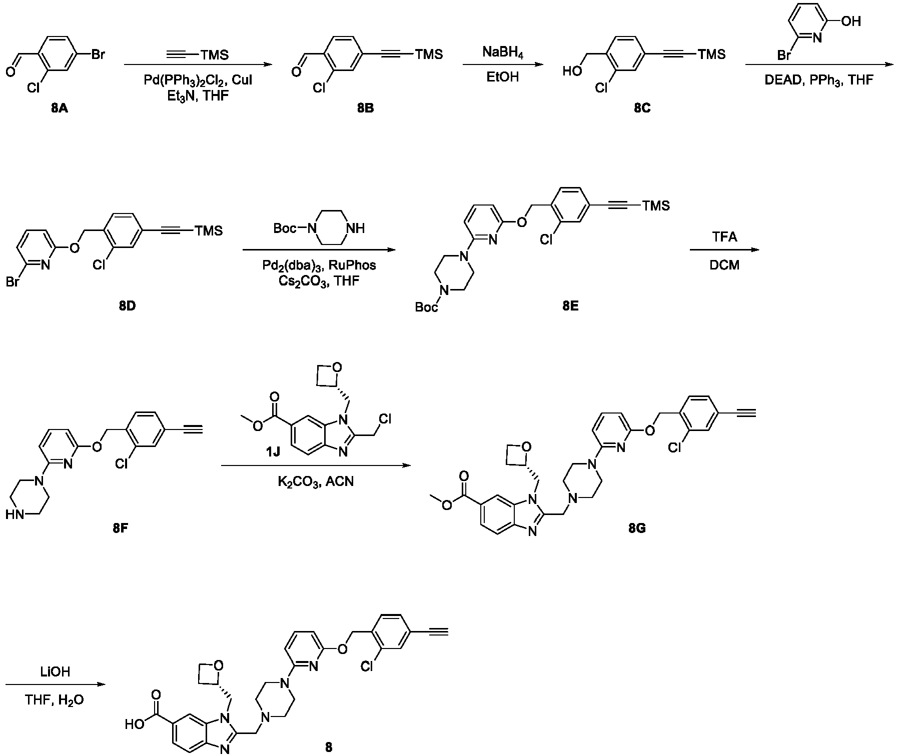

### Specific steps were as follows:

### Step 8-1: Synthesis of Compound 8D

According to the synthesis route of Example **1,** in Step **1-3,** Compound **1E** was replaced with Compound **8A,** to obtain Compound **8C;** and according to the synthesis route of Example **2,** in Step **2-1,** Compound **1G** was replaced with Compound **8C,** and at the same time, 2-chloro-6-hydroxypyridine was replaced with 2-bromo-6-hydroxypyridine, to obtain Compound **8D.** LCMS (ESI +): 394.15 (M + H)+.

### Step 8-2: Synthesis of Compound 8E

Compound **8D** (500 mg, 1.27 mmol) obtained in Step **8-1,** N-Boc piperazine (283 mg, 1.52 mmol), Pd₂(dba)₃ (58 mg, 0.064 mmol), RuPhos (59 mg, 0.13 mmol), and cesium carbonate (620 mg, 1.91 mmol) were successively added into tetrahydrofuran (10 mL). After being replaced with argon for three times, the reaction mixture was heated to 70°C and reacted for about 4 h. After the reaction solution was cooled to room temperature, it was concentrated under a reduced pressure. The crude product was purified by silica gel column chromatography, eluted with 0-15% EA/PE, and a corresponding eluate was collected and concentrated, to obtain a colorless oily substance, namely Compound **8E** (610 mg). LCMS (ESI +): 500.10 (M + H)+.

### Step 8-3: Synthesis of Compound 8

According to the synthesis route of Example **2,** in Step 2-3, Compound **2B** was replaced with Compound **8E,** to obtain Compound **8.** LCMS (ESI +): 572.10 (M + H)+.

### Example 9: Synthesis of Compound 9

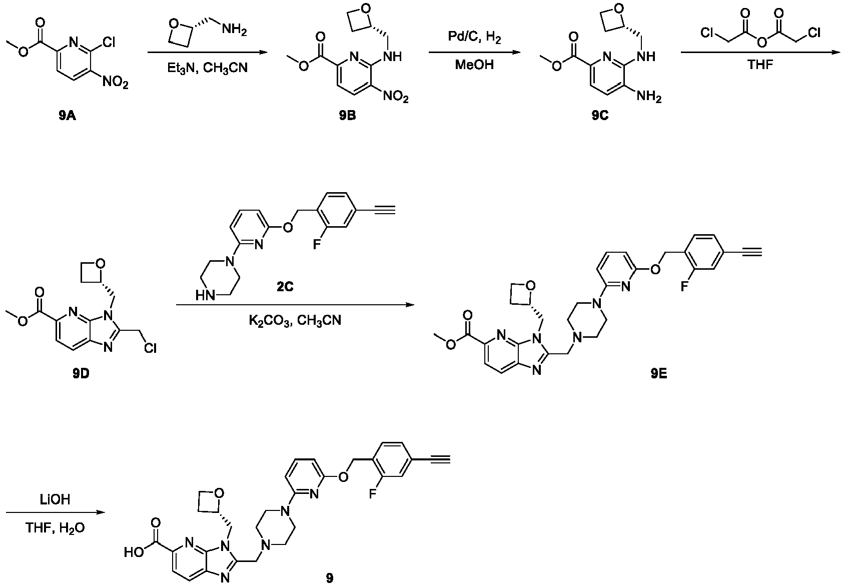

### Specific steps were as follows:

### Step 9-1: Synthesis of Compound 9B

Compound **9A** (5.00 g, 23.1 mmol), (S)-oxacyclobutane-2-methylamine (2.41 g, 27.7 mmol), and triethylamine (4.67 g, 46.2 mmol) were successively added to acetonitrile (200 mL). The reaction mixture was stirred at 50 °C for about 4 h. After the reaction solution was cooled to room temperature, it was concentrated under a reduced pressure. The crude product was purified by silica gel column chromatography, and eluted with 0-20% EA/PE. A corresponding solution was collected and concentrated, to obtain a yellow solid, namely Compound **9B** (5.2 g). LCMS (ESI +): 268.21 (M + H)+.

### Step 9-2: Synthesis of Compound 9C

Compound **9B** (4.5 g, 18.0 mmol) obtained in Step **9-1** was added into a 100 mL round-bottom flask, and dissolved in methanol (50 mL), and then Pd/C (2.0 g, 10w%, 50% water) was added. The reaction mixture was replaced with hydrogen for 3 times, and reacted for about 3 h under a hydrogen atmosphere. The reaction solution was filtered by diatomaceous earth, and a filtrate was concentrated under a reduced pressure, to obtain a yellow solid, namely Compound **9C** (3.7 g). LCMS (ESI +): 238.05 (M + H)+.

### Step 9-3: Synthesis of Compound 9D

Compound **9C** (3.7 g, 15.6 mmol) obtained in Step **9-2** was dissolved in tetrahydrofuran (50 mL), and a tetrahydrofuran (10 mL) solution of chloroacetic anhydride (2.93 g, 17.2 mmol) was added at room temperature under rapid stirring. The reaction mixture was heated to 55 °C and reacted for about 6 h. The reaction solution cooled to room temperature was poured into a mixture of ethyl acetate (150 mL) and a saturated sodium bicarbonate aqueous solution (200 mL). The solution was separated, and an organic phase was collected. The organic phase was washed with a saturated sodium chloride aqueous solution (100 mL), and dried with anhydrous sodium sulfate. After a filtration, a filtrate was concentrated under a reduced pressure. The crude product was purified by silica gel column chromatography, and eluted with 0-80% EA/PE. A corresponding solution was collected and concentrated, to obtain a light yellow solid, namely Compound **9D** (4.1 g), which was directly used for the next step. LCMS (ESI +): 296.30 (M + H)+.

### Step 9-4: Synthesis of Compound 9

According to the synthesis route of Example **2,** in Step **2-4,** Compound **1J** was replaced with Compound **9D,** to obtain Compound 9. LCMS (ESI +): 557.10 (M + H)+.

### Example 10: Synthesis of Compound 10

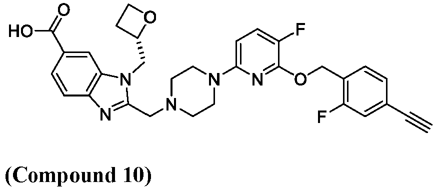

According to the synthesis route of Example **2,** in Step **2-1,** 2-chloro-6-hydroxypyridine was replaced with 6-chloro-3-fluoropyridine-2-ol, to obtain Compound **10.** LCMS (ESI +): 574.2 (M + H)+.

### Example 11: Synthesis of Compound 11

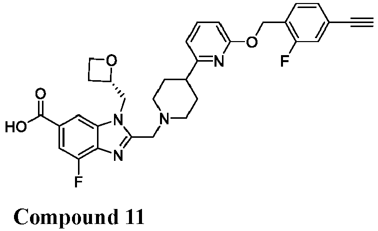

According to the synthesis route in Example **1,** in Step **1-7,** Compound **1J** was replaced with Compound **6D,** to obtain Compound **11.** LCMS (ESI +): 573.3 (M + H)+.

### Example 12: Synthesis of Compound 12

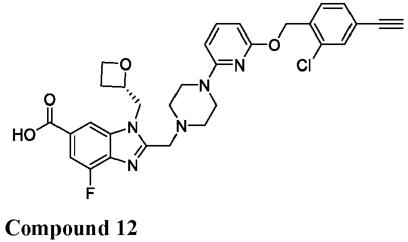

According to the synthesis route of Example **2,** in Step **2-3,** Compound **2B** was replaced with Compound **8E,** to obtain Compound **8F;** and in Step **2-4,** Compound **2C** was replaced with Compound **8F,** and Compound **1J** was replaced with Compound **6D,** to obtain Compound **12.** LCMS (ESI +): 590.11 (M + H)+.

### Example 13: Synthesis of Compound 13

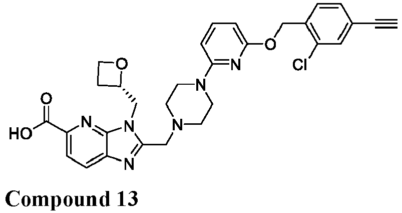

According to the synthesis route of Example **2,** in Step **2-3,** Compound **2B** was replaced with Compound **8E,** to obtain Compound **8F;** and in Step **2-4,** Compound **2C** was replaced with Compound **8F,** and Compound **1J** was replaced with Compound **9D,** to obtain Compound **13.** LCMS (ESI +): 573.21 (M + H)+.

### Example 14: Synthesis of Compound 14

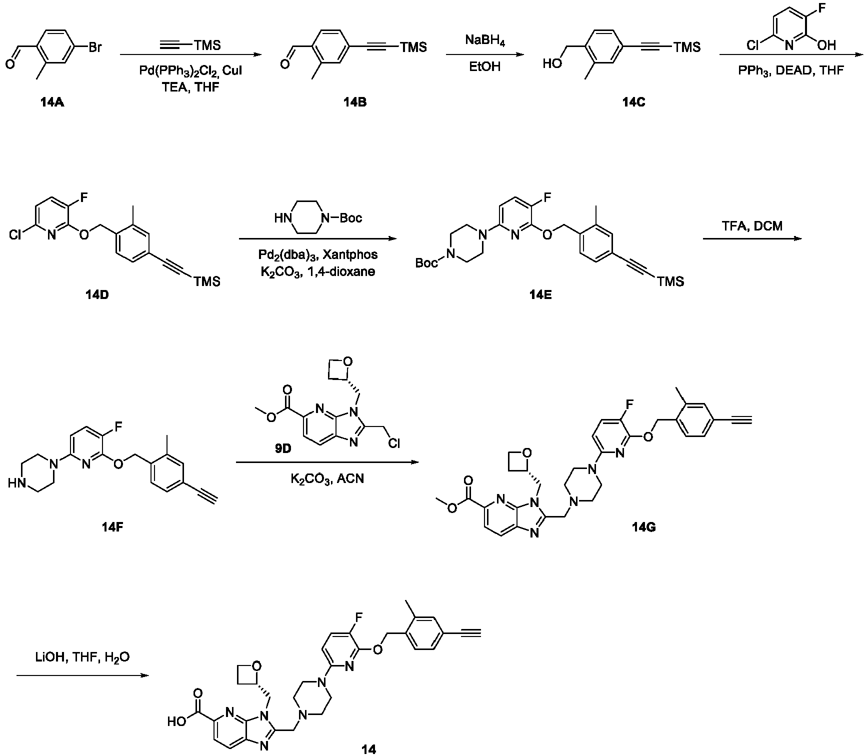

### Specific steps were as follows:

According to the synthesis route of Example **1,** in Step **1-3,** Compound **1E** was replaced with Compound **14A,** to obtain Compound **14C.**

According to the synthesis route of Example **2,** in Step **2-1,** Compound **1G** was replaced with Compound **14C,** and 2-chloro-6-hydroxypyridine was replaced with 6-chloro-3-fluoropyridine-2-ol; and in Step **2-4,** Compound **1J** was replaced with Compound **9D,** to obtain Compound **14.** LCMS (ESI +): 571.3 (M + H)+.

### Example 15: Synthesis of Compound 15

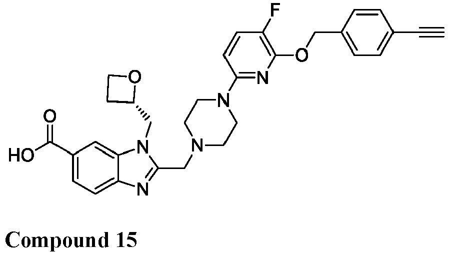

According to the synthesis route of Example **2,** in Step **2-1,** Compound **1G** was replaced with 4-(trimethylsilylacetenyl)benzyl methanol, and 2-chloro-6-hydroxypyridine was replaced with 6-chloro-3-fluoropyridine-2-ol, to obtain Compound **15.** LCMS (ESI +): 556.15 (M + H)+.

### Example 16: Synthesis of Compound 16

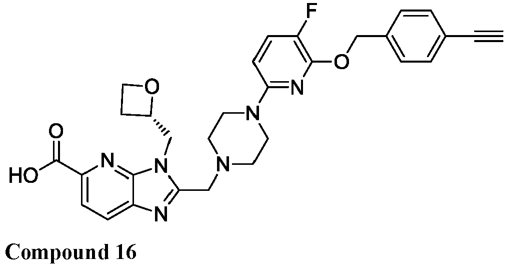

According to the synthesis route of Example **2,** in Step **2-1,** Compound **1G** was replaced with 4-(trimethylsilylacetenyl)benzyl methanol, and 2-chloro-6-hydroxypyridine was replaced with 6-chloro-3-fluoropyridine-2-ol; and in Step **2-4,** Compound **1J** was replaced with Compound **9D,** to obtain Compound 16. LCMS (ESI +): 557.31 (M + H)+.

### Example 17: Synthesis of Compound 17

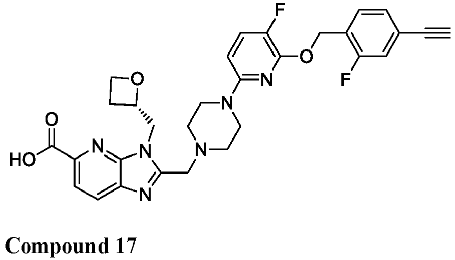

According to the synthesis route of Example **2,** in Step **2-1,** 2-chloro-6-hydroxypyridine was replaced with 6-chloro-3-fluoropyridine-2-ol, and in Step **2-4,** Compound **1J** was replaced with Compound **9D,** to obtain Compound **17.** LCMS (ESI +): 575.40 (M + H)+.

### Example 18: Synthesis of Compound 18

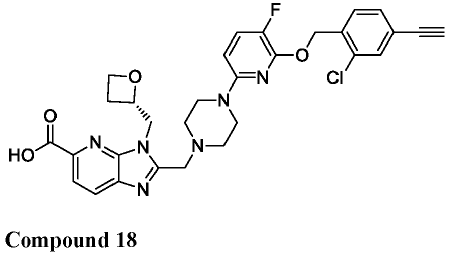

According to the synthesis route of Example **1,** in Step **1-3,** Compound **1E** was replaced with Compound **8A,** to obtain Compound **8C.** Then, according to the synthesis route of Example **2,** in Step **2-1,** Compound **1G** was replaced with Compound **8C,** and 2-chloro-6-hydroxypyridine was replaced with 6-chloro-3-fluoropyridine-2-ol; and in Step **2-4,** Compound **1J** was replaced with Compound **9D,** to obtain Compound **18.** LCMS (ESI +): 591.2 (M + H)+.

### Example 19: Synthesis of Compound 19

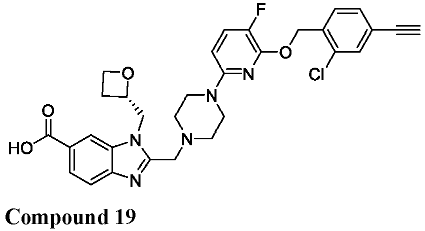

According to the synthesis route of Example **1,** in Step **1-3,** Compound **1E** was replaced with Compound **8A,** to obtain Compound **8C.** Then, according to the synthesis route of Example **2,** in Step **2-1,** Compound **1G** was replaced with Compound **8C,** and 2-chloro-6-hydroxypyridine was replaced with 6-chloro-3-fluoropyridine-2-ol, to obtain Compound **19.** LCMS (ESI +): 590.2 (M + H)+.

### Example 20: Synthesis of Compound 20

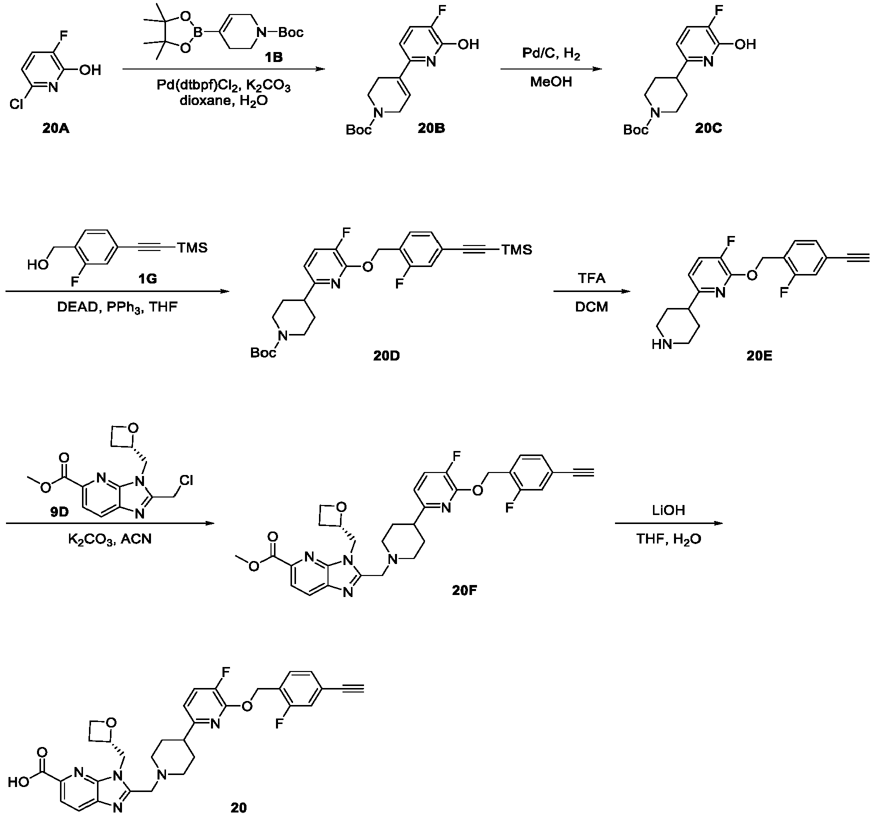

### Specific steps were as follows:

### Step 20-1: Synthesis of Compound 20B

Compound **20A** (2.00 g, 13.6 mmol), Compound **1B** (8.38 g, 27.1 mmol), Pd(dtbpf)Cl₂ (90 mg, 0.14 mmol), and potassium carbonate (7.49 g, 54.2 mmol) were successively added into a mixed solvent of dioxane (30 mL) and water (5 mL). The mixture was replaced with argon for three times, so that reaction mixture was located in an argon atmosphere, and then the temperature of the reaction mixture was raised to 100 °C and reacted for about 3 h. After the reaction solution was cooled to room temperature, it was poured into ethyl acetate (150 mL) and the pH value of the mixture was adjusted to about 7 by acetic acid. An organic phase was separated, and dried by anhydrous sodium sulfate; and after a solid was removed by filtering, a filtrate was then concentrated under a reduced pressure. The crude product was purified by silica gel column chromatography, and eluted with 0-60% EA/PE. A corresponding eluate was collected and concentrated. A light brown solid compound **20B** (1.66 g) was obtained. LCMS (ESI +): 295.21 (M + H)+.

### Step 20-2: Synthesis of Compound 20C

Compound **20B** (1.10 g, 3.74 mmol) obtained in Step **20-1** was added into a 100 mL round-bottom flask, and dissolved in methanol (25 mL), and then Pd/C (500 mg, 10w%, 50% water) was added. The reaction mixture was replaced with hydrogen for 3 times, so that the reaction mixture was reacted under a hydrogen atmosphere for about 16 h. The reaction solution was filtered by diatomaceous earth, and a filtrate was concentrated under a reduced pressure, to obtain a white solid, namely Compound **20C** (1.10 g), which was directly used for the next step. LCMS (ESI +): 297.31 (M + H)+.

### Step 20-3: Synthesis of Compound 20

According to the synthesis route of Example **1,** in Step **1-5,** Compound **1D** was replaced with Compound **20C,** and in Step **1-7,** Compound **1J** was replaced with Compound **9D,** to obtain Compound **20.** LCMS (ESI +): 574.40 (M + H)+.

### Example 21: Synthesis of Compound 21

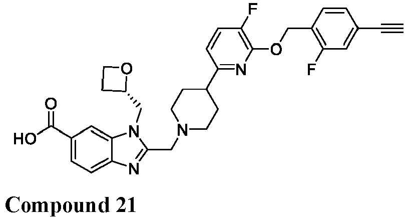

According to the synthesis route of Example **1,** in Step **1-5,** Compound **1D** was replaced with Compound **20C,** to obtain Compound **21.** LCMS (ESI +): 573.09 (M + H)+.

### Example 22: GLP-1R agonist activity determination

A GLP-1R cAMP experiment was used to determine the GLP-1R agonist activity of Compounds 1-21 of the present application and a known control medicament PF-06882961.

### 1. Reagents and consumables

### (1) Reagents

| |
|---|
| cAMP detection kit, Cisbio (Cat # 62AM4PEJ) |
| 1× HBSS, Invitrogen (Cat # 14025) |
| PF-06882961, Ningbo Benkangjishi Pharmaceutical Technology Co (CAS# 2230198-02-2) |
| Exendin-4, Hao Yuan (HY-13443A) |

### (2) Cell information

| |
|---|
| HEK293 cell line stably transfected with GLP-1R (hereinafter referred to as a GLP-1R-HEK293 cell), from WuXi AppTec, Accession No: NM_002062.4 |

### (3) Devices

| |
|---|
| OptiPlate 384, white, PerkinElmer (Cat # 6007290) |
| 384-well plate, used for Echo, Labcyte (Cat#P-05525) |
| Envision, PerkinElmer |
| Vi-cell counter, Beckman (Cat# Vi-CELL^{™} XR Cell Viability Analyzer) |

### 2. Experimental method

### 2.1 Configuration of compound source plate

An automated liquid treatment platform was used, and Compounds 1-21 of the present application, a known control medicament PF-06882961, and a positive control medicament Exendin-4 (in order to monitor experimental consistency) were diluted respectively. The initial concentration was 500 nM, the compounds were diluted according to a 4-fold gradient in a total of 10 concentrations, and the diluent was dimethyl sulfoxide.

### 2.2 Transfer of compounds

1) 100 nL of each compound was transferred to a OptiPlate 384-well plate by using an Echo liquid transfer system; and
2) the 384-well plate was centrifuged at 1000 rpm for 5 s.

### 2.3 Preparation of cell suspension

1) According to a conventional cell thawing method, a GLP-1R-HEK293 cell line was rapidly thawed in a 37 °C water bath;
2) the cell suspension was transferred to a 15 mL centrifuge tube containing 10 mL of 1×HBSS solution;
3) at room temperature, the tube was centrifuged at 1000 rpm for 5 min, to collect cells;
4) a supernatant was discarded carefully, and attention was paid to avoid sucking away the cells;
5) the centrifuge tube was flicked gently, so that the cells were loosened, and 10 mL of 1×HBSS solution was added to resuspend the cells. The cells were blown and beaten up and down by using a sterile pipette, until the cells were blown and beaten to a single-cell state;
6) the cells were counted by using a Vi-cell counter, to determine the cell concentration and determine the cell activity;
7) GLP-1R-HEK293 cells were resuspended in a cAMP detection buffer solution (from the above cAMP detection kit), and the concentration was 2.0×10⁵/mL; and
8) 10 µL of the GLP-1R-HEK293 cells was transferred into the OptiPlate 384-well plate.

### 2.4 HTRF cAMP agonist detection

1) The 384-well plate was incubated at room temperature for 20 min, then a cAMP detection reagent (from the above cAMP detection kit) was added;
2) an electric multi-channel pipette was used to add 10 µL of the cAMP detection reagent to each well;
3) the 384-well plate was covered by using a TopSeal-A film, and incubated at room temperature for 60 min; and
4) the TopSeal-A film was removed, and a signal on an Envision instrument was read.

### 3. Experimental result

GLP-1R cAMP determination results (expressed as EC50) for Compounds 1-21 of the present application and the known control medicament PF-06882961 are shown in Table 1.

**Table 1**

| **Compound number** | **EC50 (nM)** |
|---|---|
| 1 | 0.82 |
| 2 | 0.96 |
| 3 | 0.80 |
| 4 | 1.75 |
| 5 | 5.20 |
| 6 | 0.34 |
| 7 | 0.39 |
| 8 | 0.10 |
| 9 | 0.15 |
| 10 | 0.10 |
| 11 | 0.14 |
| 12 | 0.41 |
| 13 | 0.36 |
| 14 | 1.30 |
| 15 | 0.85 |
| 16 | 1.07 |
| 17 | 0.56 |
| 18 | 0.37 |
| 19 | 0.51 |
| 20 | 0.59 |
| 21 | 0.57 |
| PF-06882961 | 0.35 |

From Table 1, it may be seen that in the GLP-1R cAMP experiment, all compounds show an effective GLP-1R agonist effect, and it is indicated that Compounds 1-21 of the present application may serve as effective GLP-1R receptor agonists.

### Example 23: Oral pharmacokinetics in mice

Compounds **1, 2, 6, 7, 8, 9, 10, 11, 13, 17, 18,** and **19** of the present application and a known control medicament PF-06882961 were subjected to a pharmacokinetic test by using the following experimental procedures:

### 1. Experimental animal

Healthy male C57 mice (9 mice per compound, n=3), purchased from Shanghai Jihui Laboratory Animal Care Co., LTD, with an age of 6-8 weeks.

### 2. Method

The mice were fasted overnight before oral administration, and drunk freely, and feeding was resumed after 4 h of the administration. According to the weight of the mice weighed before the administration, the administration dose was calculated. It was intragastrically administered, and the administration dose was 10 mg/kg. At 0.083 h, 0.25 h, 0.5 h, 1 h, 2 h, 4 h, 8 h, and 24 h after the administration to test animals, about 110 µL of whole blood were respectively collected from submandibular veins of the mice by a semi-continuous method, and placed in a test tube containing K₂EDTA. The tube was placed on ice, and centrifuged within 30 min (2000 g, 5 min, 4 °C), plasma was collected, transferred to an appropriately labeled test tube, and frozen at -80 °C in a refrigerator.

### 3. Chromatography and mass spectrometry conditions

A chromatographic column was Waters ACQUITY UPLC BEH C18 (2.1×50 mm, and 1.7 µm); mobile phase A: H₂O-0.025%FA-1mM NH₄OAC, mobile phase B: ACN-0.025%FA-1mM NH₄OAC, flow rate: 0.60 mL/min, gradient elution program: start, 2% B, 0.2 min; 60% B, 0.7 min; 90% B, 1.50 min; 90% B, 2.0 min; 2% B, 2.01 min; 2.6 min, and stop; column temperature: 50 °C; and injection volume: 1 µL.

Mass spectrometry method: LC-MS/MS-49 (Triple Quad 6500+)(SCIEX, USA), the ion source was an ESI source, the detection mode was positive ion detection, and the scanning mode was a multi-reaction monitoring (MRM) mode, m/z: 446.20/321.10 Da (Glipizide, internal standard).

### 4. Preparation of plasma sample

10 µL of a plasma sample was taken and 200 µL of an internal standard working solution (Glipizide, 50 ng/mL) was added, vortex was performed for 1 min, then the mixture was centrifuged at 5800 rpm for 10 min. 70 µL of a supernatant was transferred to a 96-well plate, and 1 µL of the supernatant was taken and injected into LC-MS/MS for analysis.

### 5. Result analysis

Pharmacokinetic (PK) parameters were calculated by using Phoenix WinNonlin 7.0; and the oral pharmacokinetic parameters of the mice, including AUC, Cₘₐₓ, Tₘₐₓ, T_{1/2} and the like, were estimated by using a non-compartmental model. Results of the oral pharmacokinetic parameters of Compounds **1, 2, 6, 7, 8, 9, 10, 11, 13, 17, 18,** and **19** and PF-06882961 in the mice are shown in Table 2.

**Table 2**

| **Compound number** | **T_{1/2} h** | **Tₘₐₓ h** | **Cₘₐₓ ng/mL** | **AUC₍₀₋ₜ₎ h*ng/mL** | **AUC_{(0-∞)} h*ng/mL** | **MRT₍₀₋ₜ₎ h** | **MRT_{(0-∞)} h** |
|---|---|---|---|---|---|---|---|
| 1 | 2.2 | 0.50 | 1696.5 | 3308.0 | 3564.7 | 1.5 | 1.8 |
| 2 | 2.6 | 0.50 | 3169.1 | 6933.5 | 7008.2 | 2.8 | 3.0 |
| 6 | 3.1 | 0.25 | 2960.0 | 3587.0 | 3596.0 | 1.7 | 1.7 |
| 7 | 3.1 | 0.50 | 6173.0 | 12502.0 | 12519.0 | 2.0 | 2.0 |
| 8 | 2.3 | 1.00 | 4367.0 | 10936.0 | 10940.0 | 2.5 | 2.5 |
| 9 | 1.4 | 0.50 | 2620.0 | 3881.0 | 3923.0 | 1.3 | 1.4 |
| 10 | 2.7 | 0.50 | 4350.0 | 9778.0 | 9800.0 | 2.5 | 2.6 |
| 11 | 3.9 | 0.50 | 2033.0 | 2273.0 | 2282.0 | 1.8 | 1.9 |
| 13 | 2.1 | 0.50 | 1763.0 | 4152.0 | 4383.0 | 1.8 | 2.3 |
| 17 | 3.0 | 0.25 | 2830.0 | 5130.0 | 5136.0 | 1.9 | 1.9 |
| 18 | 2.6 | 0.50 | 1471.0 | 4133.0 | 4138.0 | 3.1 | 3.1 |
| 19 | 2.4 | 0.50 | 3300.0 | 10309.0 | 10315.0 | 2.7 | 2.7 |
| PF-06882961 | 1.4 | 0.25 | 4967.0 | 3427.0 | 3444.0 | 0.8 | 0.8 |

From Table 2, it may be seen that compared with the known control medicament (PF-06882961), Compounds **1, 2, 6, 7, 8, 9, 10, 11, 13, 17, 18,** and **19** of the present application have longer halflives (T_{1/2}) and/or higher overall exposure doses (AUC).

### Example 24: Mouse glucose tolerance test of Compound 2 in the present application

Specific steps were as follows:
1) After the arrival of experimental animals (hGLP-1R transgenic mouse B-hGLP-1R, SPF-grade mouse, male, 6-8 weeks, and purchased from Baiosaitu Jiangsu Gene Biotechnology Co., Ltd.), all experimental animals were divided into one animal per cage and fed and adapted for 7 days before the start of the experiment (during which the health status of the animals was observed). At least 3 days before the start of IPGTT, the animals were weighed and solvent (0.5% methylcellulose solution) intragastric administration and intraperitoneal saline injection were performed.
2) On the day before the experiment, the experimental animals were fasted overnight (14 h, 19:00-9:00), and at 9:00 am, the experimental animals were weighed and blood was collected from tail tips to measure the blood glucose. Based on the blood glucose and weight data, the experimental animals were evenly divided into 3 groups:
   a solvent group (namely "hGLP-1R + solvent" in Fig. 1), a 10 mg/kg PF-06882961 (namely "hGLP-1R + PF-06882961" in Fig. 1) group, and a 10 mg/kg Compound 2 (namely "hGLP-1R + Compound 2" in Fig. 1) group.
3) The grouped experimental animals were orally gavaged with 10 mL/kg 0.5% methylcellulose solution, 10 mg/kg PF-06882961, and 10 mg/kg Compound 2 (09:30 am) respectively.
4) After 15 min of the oral gavage (09:45 am), the experimental animals were intraperitoneally injected with 2 g/kg 40% dextrose solution.
5) Blood was collected by a tail tip blood sampling mode at 1 min before the injection of the glucose solution (09:44 am) and at 15 min (10:00 am), 30 min (10:15 am), 45 min (10:30 am), 60 min (10:45 am), 90 min (11:15 am), and 120 min (11:45 am) after the injection of the glucose solution, and blood glucose values were detected. Results are shown in Fig. 1.

As shown in Fig. 1, Compound **2** of the present application has a significant blood glucose reducing effect, and the overall blood glucose-reducing effect is better than PF-06882961.

Finally, it should be noted that the above examples are only used to describe the technical solutions of the present application, and not to limit it; although the present application is described in detail with reference to the aforementioned examples, it should be understood by those of ordinary skill in the art that: the technical solutions recorded in the aforementioned examples may still be modified, or some of the technical features therein are equivalently replaced; and these modifications or replacements do not make the essence of the corresponding technical solutions deviate from the spirit and scope of the technical solutions in various examples of the present application.

### Industrial Applicability

The present application provides compounds with GLP-1R receptor agonist activity, preparation methods therefor, and uses thereof. The compounds provided by the present application have excellent GLP-1R receptor agonist activity and significant blood glucose-reducing effect, have better pharmacokinetic characteristics, and provide more options for the prevention and/or treatment of GLP-1 activity related-diseases, conditions, or disorders, and thereby have broad clinical application prospects.

## Claims

1. A compound as shown in General formula (I) or a pharmaceutically acceptable salt thereof: 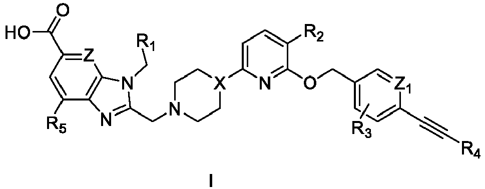 wherein,
R₁ is selected from (S)-oxacyclobutan-2-yl, 1-tetrahydrofuran-3-yl, 1-ethyl-1*H*-imidazol-4-yl, or oxazol-2-yl;
R₂ is selected from hydrogen or halogen;
R₃ is selected from hydrogen, C₁-C₆ alkyl, or halogen;
R₄ is selected from hydrogen, optionally substituted C₁-C₆ alkyl, or optionally substituted C₃-C₇ cycloalkyl;
R₅ is selected from hydrogen or halogen; and
X, Z, and Z₁ are each independently selected from -CH or nitrogen.

2. The compound or a pharmaceutically acceptable salt thereof according to claim 1, wherein,
R₁ is (*S*)-oxacyclobutan-2-yl;
R₂ is selected from hydrogen or halogen;
R₃ is selected from hydrogen, C₁-C₃ alkyl, or halogen;
R₄ is selected from hydrogen, optionally substituted C₁-C₆ alkyl, or optionally substituted C₃-C₇ cycloalkyl;
R₅ is selected from hydrogen or fluorine; and
X, Z, and Z₁ are each independently selected from -CH or nitrogen.

3. The compound or a pharmaceutically acceptable salt thereof according to claim 1 or 2, wherein,
R₁ is (*S*)-oxacyclobutan-2-yl;
R₂ is selected from hydrogen or fluorine;
R₃ is selected from hydrogen, methyl, fluorine, or chlorine;
R₄ is selected from hydrogen, hydroxymethyl, or cyclopropyl;
R₅ is selected from hydrogen or fluorine;
X and Z are each independently selected from -CH or nitrogen; and
Z₁ is -CH.

4. The compound or a pharmaceutically acceptable salt thereof according to claim 1 or 2, wherein it is selected from:

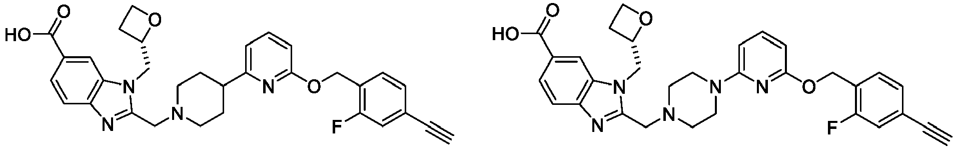

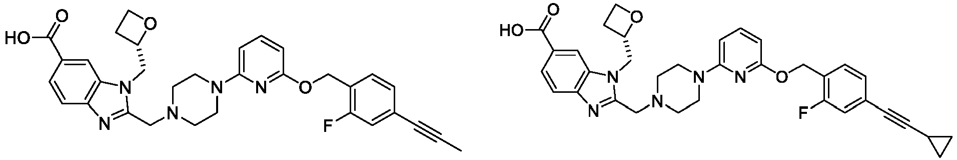

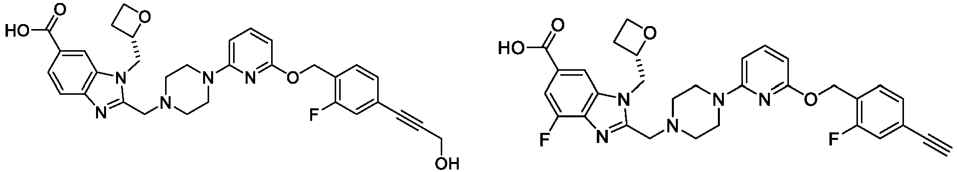

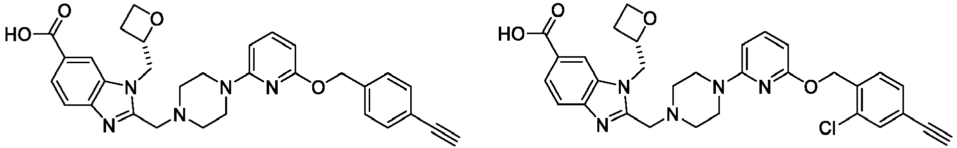

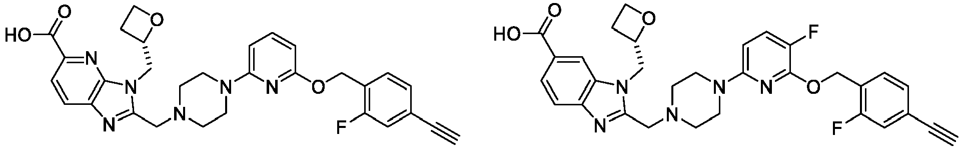

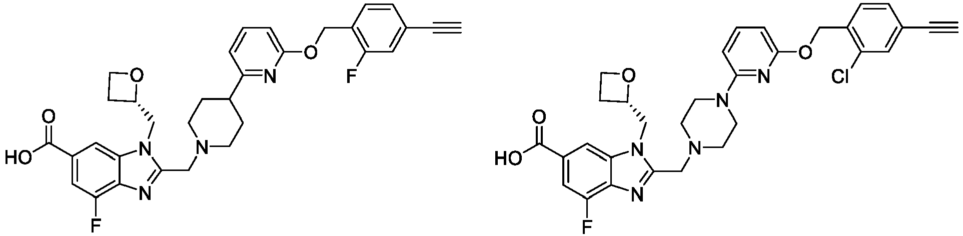

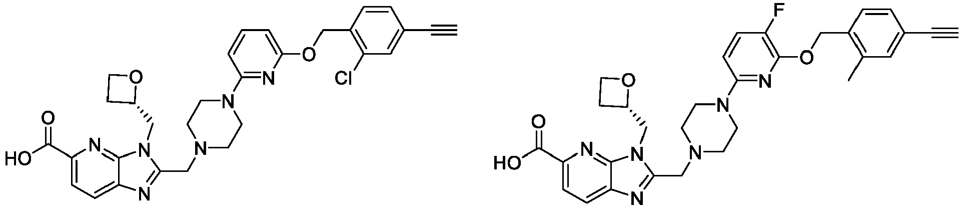

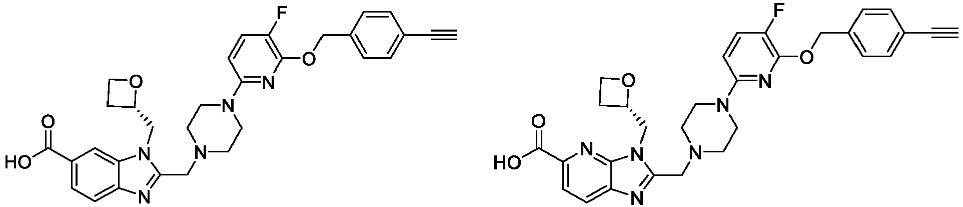

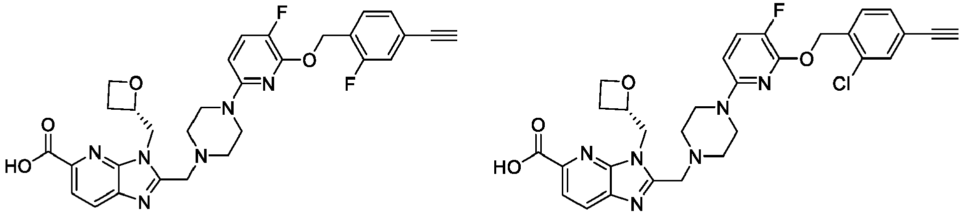

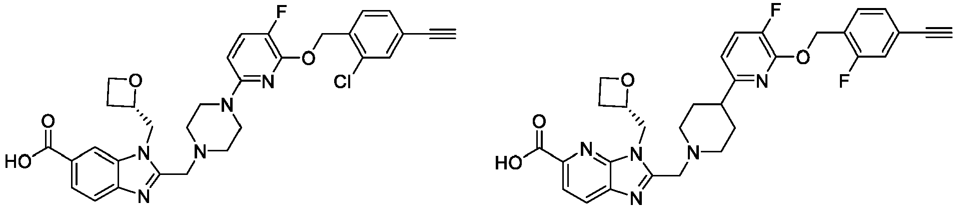

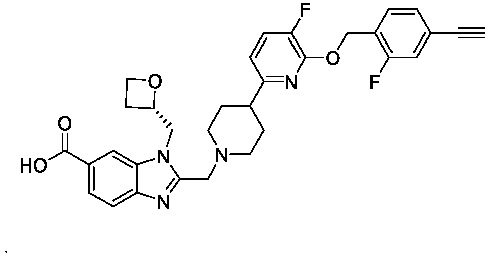

5. A pharmaceutical composition comprising the compound or a pharmaceutically acceptable salt thereof according to any one of claims 1 to 4 and a pharmaceutically acceptable carrier, diluent, and/or excipient.

6. Use of the compound or a pharmaceutically acceptable salt thereof according to any one of claims 1 to 4 in the preparation of a glucagon-like peptide-1 receptor agonist.

7. Use of the compound or a pharmaceutically acceptable salt thereof according to any one of claims 1 to 4 or the pharmaceutical composition according to claim 5 in the preparation of a medicament for preventing and/or treating diseases, conditions, or disorders related to glucagon-like peptide-1 activity, wherein the diseases, conditions, or disorders can be regulated or treated by activating a glucagon-like peptide-1 receptor.

8. The use according to claim 7, wherein the diseases, conditions, or disorders are selected from the group consisting of the followings:
type 1 diabetes, type 2 diabetes mellitus, prediabetes, idiopathic type 1 diabetes mellitus, latent autoimmune diabetes in adults, early-onset type 2 diabetes, young-onset atypical diabetes, maturity-onset diabetes of the young, malnutrition-related diabetes mellitus, gestational diabetes mellitus, hyperglycemia, insulin resistance, glucose intolerance, diabetic neuropathy, diabetic nephropathy, kidney disease, diabetic retinopathy, adipocyte dysfunction, visceral adipocyte accumulation, sleep apnoea, obesity, eating disorder, weight gain caused by the use of other medicaments, sugar fanatic, dyslipidemia, hyperinsulinemia, non-alcoholic fatty liver disease, non-alcoholic steatohepatitis, fibrosis, sclerosis, hepatocellular carcinoma, cardiovascular disease, atherosclerosis, coronary artery disease, peripheral vascular disease, hypertension, endothelial dysfunction, injured vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipidemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome X, premenstrual syndrome, angina pectoris, thrombosis, atherosclerosis, transient ischemic attack, vascular restenosis, poor glucose metabolism, impaired fasting glucose condition, hyperuricemia, gout, erectile dysfunction, skin and connective tissue abnormalities, psoriasis, foot ulcer, ulcerative colitis, hyper-Apo B lipoproteinemia, Alzheimer's disease, schizophrenia, cognitive impairment, inflammatory bowel disease, short bowel syndrome, Crohn's disease, colitis, irritable bowel syndrome, and polycystic ovary syndrome;
preferably, the diseases are type 2 diabetes mellitus or the following conditions related thereto: hyperglycemia, insulin resistance, glucose intolerance, diabetic neuropathy, diabetic nephropathy, diabetic retinopathy, obesity, dyslipidemia, hypertension, hyperinsulinemia, or non-alcoholic fatty liver disease.
